# EUROPÄISCHE PATENTSCHRIFT

(11) **EP 0 548 027 B1**
(45) Veröffentlichungstag und Bekanntmachung des Hinweises auf die Patenterteilung: **12.02.1997**
(21) Anmeldenummer: 92811003.0
(22) Anmeldetag: 15.12.1992
(51) Int. Cl.: G01J 3/10, G01J 3/28, A61B 5/00, G01N 33/49

(54) **Einrichtung zur spektralphotometrischen Analyse**
Apparatus for spectro photometric analysis
Appareil pour l'analyse spectrométrique

(30) Priorität: 17.12.1991 CH 3734/91
(43) Veröffentlichungstag der Anmeldung: 23.06.1993
(73) Patentinhaber: AVL Medical Instruments AG, 8207 Schaffhausen (CH)
(72) Erfinder: Hatschek,Rudolf Alexander,Dr., CH-1700 Fribourg 5 (CH)
(74) Vertreter: Krause, Walter, Dr. Dipl.-Ing.

(56) Entgegenhaltungen:
- EP-A- 0 290 275
- EP-A- 0 358 809
- WO-A-91/11136
- DE-A- 3 215 879
- US-A- 3 638 640
- PATENT ABSTRACTS OF JAPAN vol. 4, no. 86 (P-16)(568) 20. Juni 1980 & JP-A-55 048 624 (HITACHI SEISAKUSHO) 7. April 1980 & JP-A-55 048 624 ( HITACHI SEISAKUSHO ) 7 April 1980.

## Beschreibung

Die Erfindung betrifft eine Einrichtung zur spektralfotometrischen Analyse eines Materials, mit einer Lichtquelle, einem Dispersionselement zur spektralen Aufteilung von Licht, einem Lichtempfänger mit einer Anzahl fotoelektrischer Wandler und einer elektrisch mit dem Lichtempfänger verbundenen Auswertungsvorrichtung, wobei der Lichtempfänger zur Erzeugung von elektrischen Signalen ausgebildet ist, die ein Maß für die Lichtintensität von in seine Wandler gestrahltem Licht geben.

Die genannte Einrichtung ist beispielsweise vorgesehen, um eine quantitative Analyse eines in einer als Lösungs- und/oder Suspensionsmittel dienenden Flüssigkeit gelösten und/oder suspendierten Materials durchzuführen. Das Material kann zum Beispiel aus einem einzigen Stoff oder aus einer Mischung von mehreren Stoffen bestehen. Die Einrichtung und das Verfahren sollen zum Beispiel die Messung einer Gassättigung der Hämoglobine von Blut ermöglichen.

Eine aus der DE-A-32 15 879 bekannte Einrichtung für die spektralphotometrische Messung der Sauerstoffsättigung von Blut besitzt als Lichtquelle eine Xenonlampe. Die bekannte Einrichtung besitzt eine zum Einführen in ein Blutgefäss bestimmte Kanüle und ein durch ein Beugungsgitter gebildetes Dispersionselement, um das Licht spektral zu zerlegen. Ferner hat diese Einrichtung einen Lichtempfänger mit einer Diodenzeile. Deren Fotodioden sind dabei derart angeordnet, dass jede vom Dispersionselement in eine bestimmte Richtung abgelenktes Licht empfangen kann. Die Diodenzeile ist mit einer Auswertungsvorrichtung verbunden, die einen Rechner besitzt. Dieser ermittelt bei einer Analyse aus der Lage der Lichtintensitätsmaxima die Sauerstoffsättigung des Blutes.

Handelsübliche Xenonlampen erzeugen ein kontinuierliches Spektrum, dem Spektrallinien überlagert sind. Gemäss der Einleitung der DE-A-32 15 879 soll es (für die Bestimmung der Sauerstoffsättigung) zudem günstiger sein, wenn im interessierenden Wellenlängenbereich der gesamte Spektralverlauf vorhanden ist. Die Ermittlung der Lage der Lichtintensitätsmaxima eines kontinuierlichen Lichtspektrums ermöglicht jedoch nur eine grobe, sehr ungenaue Bestimmung der Sauerstoffsättigung. Im übrigen ist in der DE-A-32 15 879 nicht näher beschrieben, wie die Lichtwellenlängen den einzelnen Fotodioden zugeordnet sind. Es ist jedoch anzunehmen, dass gleich wie bei aus der Praxis bekannten, eine Diodenzeile aufweisenden Spektralphotometern jeder Fotodiode für alle durchzuführenden Analysen ein und dieselbe Lichtwellenlänge fest zugeordnet ist. Damit eine solche Einrichtung eine hohe Auflösung und Messgenauigkeit ermöglicht, müssen sich das Dispersionselement, die Fotodioden und weitere, den Verlauf der Lichtstrahlen beeinflussende Bauteile sehr genau in den vorgesehenen Positionen befinden und dauernd in diesen bleiben. Die Herstellung und einer solchen Einrichtung erfordert daher eine hohe Präzision und ist entsprechend teuer. Ferner besteht die Gefahr, dass äussere Einwirkungen - wie Erschütterungen sowie Temperatur- und Feuchtigkeitsände- rungen - die bei der Herstellung einer Einrichtung festgelegten Positionen des Dispersionselementes, der Fotodioden und sonstiger Teile verändern, so dass auch die Wellenlänge des zu einer bestimmten Fotodiode gelangenden Lichtes ändert. Man mag zwar versuchen, die störenden Einflüsse von Erschütterungen durch eine besonders stabile Ausbildung der Einrichtung und durch deren Ausrüstung mit Schwingungsdämpfern und dergleichen zu reduzieren. Des weitern kann an sich vorgesehen werden, die Einrichtung mit Vorrichtungen zur Konstanthaltung der Temperatur und Luftfeuchtigkeit auszurüsten. Massnahmen solcher oder ähnlicher Arten verursachen jedoch eine beträchtliche Verteuerung der Einrichtung und machen diese gross und schwer. Ferner ist eine solche Einrichtung kaum noch für einen für viele Verwendungen erwünschten, mobilen und/oder transportablen Einsatz geeignet, bei welchem sie nach Bedarf an verschiedene Orte gebracht und an diesen benutzt werden kann.

Die JP-A-Sho-55-48 624 betrifft eine Eichvorrichtung zum Eichen einer Spektralanalyse-Einrichtung. Die Eichvorrichtung besitzt eine Quecksilberlampe zur Erzeugung von Licht mit einem Linienspektrum. Dieses Licht wird beim Eichen anstelle des bei einer Analyse von einer anderen Lichtquelle erzeugten Lichtes über eine Lichtumschaltvorrichtung mit einem schwenkbaren Reflektor zum Dispersionselement der Spektralanalyse-Einrichtung gestrahlt. Die Eichvorrichtung besitzt ferner einen verstellbaren Lichtempfänger, der beim Eichen entlang der Fokusfläche des Dispersionselements bewegt wird.

Die aus der JP-A-Sho-55-48 624 bekannte Einrichtung hat den Nachteil, dass sie nebst den für die Analyse benötigten Komponenten zum Eichen noch mehrere zusätzliche Komponenten, insbesondere eine Quecksilberlampe, eine Lichtumschaltvorrichtung, einen verstellbaren Lichtempfänger und Mittel zur Bestimmung der Position des letzteren aufweist, wodurch der Platzbedarf und die Kosten der Einrichtung erhöht werden. Ferner sind der schwenkbare Reflektor der Lichtumschaltvorrichtung, der verstellbare Lichtempfänger und die Mittel zur Positionsbestimmung des letzteren störanfällig und empfindlich auf Erschütterungen. Da das zum Eichen dienende und das für eine Analyse benutzte Licht von verschiedenen Stellen und entlang von verschiedenen Strahlenwegen zum Dispersionselement gestrahlt wird, können die Wellenlängen des vom Letzteren beim Eichen erzeugten Spektrums und des bei der Analyse erzeugten Spektrums zudem möglicherweise gegeneinander verschoben sein, wodurch die Eichung ungenau wird.

Die Druckschritt WO-A-91/11136 offenbart eine Vorrichtung zur spektraphotometrischen Analyse nach dem Oberbegriff des Hauptanspruchs 1. Eine ähnliche Vorrichtung ist auch aus der EP-A-0 358 809 bekannt.

Der Erfindung liegt daher die Aufgabe zugrunde, Nachteile der bekannten Einrichtung zur spektralphotometrischen Analyse auszuschalten. Dabei sollen insbesondere eine Einrichtung geschaffen werden, die ermöglicht, bei einfachem, keine übermäßige Präzision erforderndem, kostengünstigen Aufbau der Einrichtung eine hohe Messgenauigkeit zu erzielen und diese aufrecht zu erhalten. Ferner soll die Einrichtung vorzugsweise problemlos von Ort zu Ort transportierbar sowie am jeweiligen Standort benutzbar sein, ohne daß durch den Transport die Genauigkeit beeinträchtigt wird.

Diese Aufgabe wird erfindungsgemäß durch eine Einrichtung mit den Merkmalen des Anspruches 1 gelöst.

Vorteilhafte Weiterbildungen der Einrichtung gehen aus den abhängigen Ansprüchen 2 bis 5 hervor.

Hier sollen noch Anmerkungen zu einigen zum Teil vorgängig schon benutzten sowie im nachfolgenden Beschreibungsteil und in den Ansprüchen vorkommenden Begriffen angebracht werden. Zum Begriff "Licht" wird darauf hingewiesen, dass dieser sowohl sichtbares als auch unsichtbares - d.h. infrarotes und ultraviolettes - Licht umfassen soll, soweit nichts anderes angegeben wird.

Die Lichtwellenlänge ist bekanntlich gleich dem Quotienten Lichtgeschwindigkeit durch Lichtfrequenz und also umgekehrt proportional zur Lichtfrequenz. Der Begriff "Lichtwellenlänge" soll daher in den Ansprüchen und in der Beschreibung - soweit nichts anderes angegeben wird - auch den Begriff "Lichtfrequenz" umfassen bzw. durch diesen ersetzbar sein. Zum Merkmal, dass jedem Wandler eine Lichtwellenlänge zugeordnet werden kann, ist anzumerken, dass dieses Merkmal auch umfassen soll, dass jedem Wandler ein gewisser, von den Abmessungen der Wandler abhängiger Bereich der Lichtwellenlänge oder der Lichtfrequenz zugeordnet werden kann, soweit nichts anderes angegeben wird und soweit sich kein Widerspruch ergibt.

Wie schon beschrieben, kann ein quantitativ zu analysierendes Material in einer Flüssigkeit gelost und/oder suspendiert sein. Die Einrichtung kann zum Beispiel ausgebildet sein, um die Anteile von verschiedenen in den roten Blutkörperchen enthaltenen Hämoglobinderivaten zu ermitteln. Die Einrichtung kann zum Beispiel ermöglichen, die Anteile von Oxyhämoglobin, Desoxyhämoglobin, CO-Hämoglobin sowie eventuell noch Methhämoglobin und/oder andern Hämoglobinderivaten zu ermitteln. Aus den Anteilen von Oxyhämoglobin und/oder CO-Hämoglomin kann dann zum Beispiel die Sauerstoffsättigung und/oder die Kohlenmonoxidsättigung des Blutes ermittelt werden. Ferner kann vorgesehen werden, zusätzlich oder anstelle der quantitativen Analyse von Hämoglobinderivaten die Anteile von andern Bestandteilen von Blut zu ermitteln. Die Einrichtung kann selbstverständlich ausgebildet sein, um anstelle von Blut andere Lösungen und/oder Dispersionen zu analysieren, in der das zu untersuchende Material gelöst und/oder dispergiert ist.

Gemäss der Erfindung ist die Lichtquelle der Einrichtung als Spektrallichtquelle ausgebildet, um Licht zu erzeugen, dessen Spektrum mindestens im wesentlichen - und im bevorzugten Idealfall vollkommen ausschliesslich - aus Spektallinien besteht. Bei der spektralen Aufteilung des Lichtes durch das Dispersionselement entstehen dann mehrere je das Licht einer eine Spektrallinie enthaltende Lichtstrahlen oder - genauer gesagt - Lichtstrahlenbündel, die zu den verschiedenen Wandlern des Lichtempfängers gelangen.

Die Lichtquelle ist also als Spektrallichtquelle ausgebildet und besitzt vorzugsweise eine Spektrallampe, nämlich eine Niederdruck-Gasentladungslampe. Diese kann zum Beispiel ein Edelgas, wie Neon, Argon, Krypton oder Xenon oder eventuell zwei oder noch mehr dieser Gase enthalten. Ferner kann eventuell eine Lampe vorgesehen werden, die als Gas zusätzlich zu mindestens einem Edelgas oder anstelle eines solchen mindestens einen Metalldampf - wie zum Beispiel Quecksilberdampf - enthält und ebenfalls als Niederdruck-Entladungslampe ausgebildet ist. Zum Begriff "Niederdruck-Entladungslampe" sei angemerkt, dass der Gas- und/oder Dampfdruck in einer solchen normalerweise höchstens 5 kPa und beispielsweise höchstens 2 kPa beträgt. Die Lichtquelle besteht vorzugsweise aus einer einzigen Lampe. Es besteht jedoch die Möglichkeit, eine Lichtquelle mit zwei oder noch mehr Lampen vorzusehen, die verschiedene Gase - zum Beispiel Neon bzw. Argon - enthalten. Das von den verschiedenen Lampen erzeugte Licht kann zum Beispiel mit Hilfe von Lichtleitern oder andern Mitteln in ein Strahlenbündel vereinigt werden.

Die Lichtquelle ist zweckmässigerweise derart ausgebildet, dass sie Licht mit Wellenlängen erzeugt, deren Werte und Abstände eine gute Analyse des zu analysierenden Materials ermöglichen. Bei der Durchführung einer Analyse kann entweder das ganze Spektrum des von der Lichtquelle erzeugten Lichtes oder nur ein Teil dieses Spektrums tatsächlich verwertet werden. Das tatsächlich für die Analyse verwertete Licht enthält vorzugsweise mindestens 5 Spektrallinien und beispielsweise mindestens 10 Spektrallinien. Für die Ermittlung der Anteile der verschiedenen Hämoglobinderivate kann zum Beispiel ein sich etwa von 530 nm bis ungefähr 620 nm oder bis höchstens 750 nm erstreckender Teil des Neon-Linienspektrums verwendet werden.

Das Dispersionselement kann zum Beispiel durch ein lichtdurchlässiges Prisma oder durch ein Beugungsgitter, insbesondere ein holographisches Beugungsgitter gebildet sein. Ein Prisma ergibt eine verhältnismässig grosse Lichtstärke. Ein Beugungsgitter ergibt normalerweise eine kleinere Lichtstärke, hat aber dafür den Vorteil, dass die Verteilung der entstehenden Lichtstrahlen mindestens annähernd oder genau linear mit der Lichtwellenlänge verknüpft ist.

Der Lichtempfänger besteht zum Beispiel aus einer integrierten Schaltung mit einer Anzahl von fotoelektrischen, die Messung der Lichtintensität ermöglichenden Wandler, könnte aber auch aus separaten Wandlern gebildet sein. Die Wandler können aus Fotohalbleitern, beispielsweise Fotodioden, bestehen. Die Wandler können in einer geraden oder eventuell leicht gebogenen Reihe angeordnet sein und zusammen einen "Array" bilden. Der Lichtempfänger kann zwischen den aktiven, die Messung der Lichtintensität ermöglichenden Zonen der einander benachbarten Wandler auch inaktive Zonen haben. Die Einrichtung ist beispielsweise derart ausgebildet, dass jeder zum Lichtempfänger gelangende Lichtstrahl breiter als die Breite einer allfälligen inaktiven Zone ist. Jeder Wandler kann beim Messen Licht eines gewissen Wellenlängenbereichs empfangen. Der bei einer Messung vom Dispersionselement für eine bestimmte Spektrallinie zum Lichtempfänger gestrahlte Lichtstrahl kann abhängig von der Ausbildung der Einrichtung und der Richtung des betreffenden Lichtstrahls nur in einen einzigen Wandler oder in eine Gruppe von zwei oder mehr einander benachbarter Wandler gelangen. Die Einrichtung ist vorzugsweise derart ausgebildet, dass zwischen jedem Paar von Wandlern oder Wandler-Gruppen, die Licht von zwei einander benachbarten Spektrallinien empfangen, jeweils mindestens ein Wandler vorhanden ist, der kein Licht empfängt.

Die Einrichtung kann zusätzlich zum Dispersionselement unterschiedlich ausgebildete Lichtführungsmittel und Sensormittel aufweisen, um von der Lichtquelle erzeugtes Licht zu einem zu analysierenden, beispielsweise in einer Flüssigkeit gelösten und/oder suspendierten Material zu führen, mit diesem in Wechselwirkung zu bringen und vom Material zum Dispersionselement zu führen. Die Lichtführungsmittel können zum Beispiel mindestens eine Fokussiervorrichtung mit mindestens einer Linse und/oder mindestens einer Blende besitzen. Die Lichtführungsmittel können ferner einen Strahlenteiler und eventuell mindestens einen Lichtleiter und/oder mindestens einen Umlenkreflektor aufweisen.

Bei einer vorteilhaften Ausgestaltung einer für die Ermittlung von Hämoglobinderivaten und eventuell andern Bestandteilen von biologischen Flüssigkeiten vorgesehenen Einrichtung kann diese zum Beispiel mit Lichtführungsmitteln und Sensormitteln ausgerüstet sein, die einen Sensor oder Messkopf mit Lichtstrahlungs- und Lichtaufnahmemitteln besitzen, um Licht in ein biologisches Objekt, zum Beispiel einen Organismus, einzustrahlen und um beispielsweise durch Rückstreuung wieder aus dem besagten Objekt herausgestrahltes Licht aufzufangen. Derartige Lichtstrahlungs- und Lichtaufnahmemittel können dann beispielsweise über flexible Lichtleiter mit den restlichen Teilen der Einrichtung verbunden sein und ermöglichen so eine nicht-invasive Analyse von einem sich in einem Organismus oder sonstigen Objekt befindenden Material.

Die Einrichtung kann ferner derart ausgebildet sein, dass die zu analysierende Probe auf einen das benutzte Licht durchlassenden oder reflektierenden Träger aufgebracht und dann mit diesem durchdringendem bzw. von diesem reflektiertem Licht analysiert werden kann. Des weitern können die Lichtführungsmittel der Einrichtung eine Sonde aufweisen, die in eine zu untersuchende Lösung und/oder Suspension eingetaucht und dann Licht durch einen Bereich von dieser hindurchstrahlen kann.

Bei einer bevorzugten Ausgestaltung ist vorgesehen, zusätzlich zu einer Analysemessung, bei welcher vom zu analysierenden Material beeinflusstes Licht spektral aufgeteilt wird, noch eine Referenzmessung durchzuführen, bei welcher mit der gleichen Lichtquelle Licht erzeugt wird wie bei der Analysemessung, so dass das für die Referenzmessung erzeugte Licht das gleiche Linienspektrum hat, wie das für die Analysemessung erzeugte Licht. Das bei der Referenzmessung erzeugte Licht wird dann durch das Dispersionselement spektral aufgeteilt, ohne dass es vorher durch das zu analysierende Material beeinflusst wurde. Wenn die Einrichtung zum Beispiel ausgebildet ist, um eine nicht-invasive Analyse von in Blutkörperchen enthaltenen Hämoglobinen durchzuführen, können die Lichtstrahlungs- und Lichtaufnahmemittel bei der Analysemessung in der schon beschriebenen Weise an einem Organismus angeordnet und für die Referenzmessung optisch in Verbindung mit einer Lichtumlenkvorrichtung gebracht werden. Diese kann dann ausgebildet sein, um von den Lichtstrahlungsmitteln ausgestrahltes Licht zu den Lichtaufnahmemitteln zu lenken, ohne dass dieses Licht in Wechselwirkung mit Blut gelangt. Das bei der Referenzmessung benützte Licht braucht dann auch in keine andere Flüssigkeit zu gelangen.

Normalerweise ist es vorteilhaft, für jede Analysemessung mindestens eine Referenzmessung durchzuführen. Die Einrichtung kann zu diesem Zweck derart ausgebildet sein, dass vor und/oder nach einer Analysemessung eine dieser zugeordnete Referenzmessung durchgeführt werden kann. Falls in kurzen Zeitabständen zwei oder mehrer Analysemessungen durchgeführt werden, kann jedoch eventuell vorgesehen werden, für eine solche Gruppe von Analysemessungen nur eine einzige Referenzmessung durch zuführen und die bei dieser ermittelten Messwerte für die ganze Gruppe von Analysemmessungen zu verwerten.

Das erzeugte Linienspektrum und dessen für eine Analyse verwerteter Bereich kann derart an die Art der Analyse angepasst werden, dass der verwertete Bereich des Linienspektrums Spektrallinien mit für die betreffenden Analyse günstigen Wellenlängen enthält. Man kann daher erreichen, dass nur relativ wenig Licht mit für die betreffende Analyse nicht benötigten Wellenlängen erzeugt wird und zum Lichtempfänger gelangt. Dies ergibt den Vorteil, dass Randeffekte und sonstige Störeffekte der verschiedenen optischen Komponenten, die Streuung des Lichtes durch Luft und ähnliche Effekte die mit dem Lichtempfänger gemessene, spektrale Verteilung der Lichtintensität höchstens wenig beeinflussen.

Die Erzeugung und Verwendung von Licht mit einem aus Spektallinien bestehenden Spektrum ergibt den Vorteil, dass die Auswertungsvorrichtung bei einer Analyse, d.h. bei einer Referenzmessung und/oder einer Analysemessung mindestens für einige der Licht empfangenden Wandler aufgrund mindestens eines vorgegebenen Kriteriums und/oder gemäss mindestens einer Vorschrift auf relativ einfache Weise und zuverlässig die Spektrallinie des betreffenden Lichtes bzw. Lichtstrahls identifizieren kann. Dadurch kann auch die Wellenlänge des zu jedem der genannten Wandler gelangenden Lichtes eindeutig und fehlerfrei ermittelt werden. Dies ermöglicht wiederum mindestens jedem bei den Messungen zur Verwertung vorgesehenes Licht empfangenden Wandler und zum Beispiel überhaupt jedem Wandler eine Lichtwellenlänge zuzuordnen. Dadurch kann gewährleistet werden, dass die einem Wandler zugeordnete Lichtwellenlänge bei jeder Analyse identisch ist mit der tatsächlichen Lichtwellenlänge des bei der Analyse in den betreffenden Wandler gestrahlten Lichtes. Dies kann insbesondere auch ohne übermässige Präzision bei der Ausbildung und Anordnung des Dispersionselementes, des Lichtempfängers und anderer den Verlauf der Lichtstrahlen beeinflussender Lichtführungsmittel gewährleistet werden. Ferner kann die genaue Zuordnung eines Wandlers zur Lichtwellenlänge des Lichtes auch dann problemlos gewährleistet werden, wenn gewisse Elemente wegen Temperatur- oder Feuchtigkeitsänderungen oder wegen bei einem Transport erfolgten Erschütterungen ihre bezüglich einander eingenommenen Positionen ein wenig geändert haben. Die Einrichtung kann daher preisgünstig, klein, leicht sowie entsprechend gut transportierbar hergestellt werden und trotzdem eine hohe Genauigkeit ermöglichen.

Die Einrichtung gemäß der Erfindung wird anschließend anhand in der Zeichnung dargestellter Ausführungsbeispiele erläutert. In der Zeichnung zeigt die
- Fig. 1: eine schematisierte Darstellung einer Einrichtung zur spektralphotometrischen Analyse, von in einem Körper zurückgestreuten Licht,
- Fig. 2: ein Diagramm zur Veranschaulichung der Auswertung der Messungen und
- Fig. 3: Teile einer Variante einer Einrichtung zur Analyse von in einem Körper zurückgestreuten Licht.

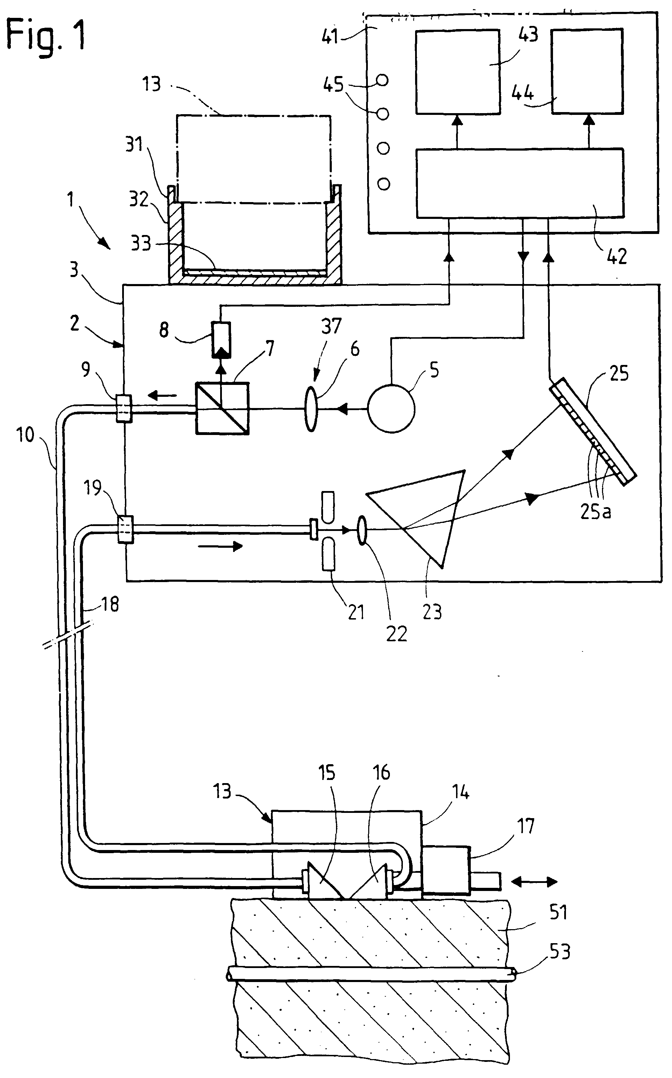

Die in der Fig. 1 ersichtliche Einrichtung 1 zur spektralphotometrischen Analyse besitzt ein Gerät 2 mit einem Gehäuse 3. In diesem ist eine Lichtquelle 5 angeordnet, die eine Spektrallampe, nämlich eine mindestens ein Edelgas enthaltende Niederdruck-Gasentladungslampe aufweist. Ferner ist eine optische Fokussiervorrichtung 6 mit mindestens einer Linse und ein Strahlenteiler 7 vorhanden. Dieser besitzt einen Lichteinlass und zwei Lichtauslässe. Dem einen von diesen ist ein Lichtsensor 8 zugewandt, der zum Beispiel aus einer Fotodiode besteht. Der andere Lichtauslass des Strahlenteilers 7 ist optisch über eine Durchführung und/oder trennbare Kupplung 9 und einen flexiblen Lichtleiter 10 mit einem optischen Sensor oder Messkopf 13 verbunden, der zumindest bei der noch näher beschriebenen Analysemessung ausserhalb des Gehäuses 3 des Gerätes 2 in Abstand von diesem angeordnet werden kann.

Der Sensor oder Messkopf 13 besitzt ein Gehäuse 14, in welchem optisch mit dem bereits erwähnten Lichtleiter 10 verbundene Lichtstrahlungsmittel 15 und Lichtaufnahmemittel 16 angeordnet sind. Die Lichtstrahlungs- und Lichtaufnahmemittel weisen zum Beispiel je einen Umlenkreflektor auf, der aus einem lichtdurchlässigen, bei der geneigten Fläche total-reflektierenden und/oder verspiegelten Prisma besteht, könnten aber auch aus metallischen Reflektoren gebildet sein. Der Sensor oder Messkopf 13 kann ferner noch eine Stellvorrichtung 17 mit einem beispielsweise manuell bewegbaren Stellelement aufweisen, so dass die Lichtaufnahmemittel 16 schrittweise oder kontinuierlich verschoben und dadurch ihr Abstand von den Lichtstrahlungsmitteln 15 verändert werden kann.

Die Lichtaufnahmemittel 16 sind optisch über einen flexiblen Lichtleiter 18 und eine am Gehäuse 3 angeordnete Durchführung und/oder trennbare Kupplung 19 mit einer im Gehäuse 3 angeordneten, eine schlitzförmige Öffnung begrenzenden Blende 21 verbunden. Die beiden Lichtleiter 10, 18 können im Querschnitt rund oder ungefähr rechteckförmig sein und weisen zum Beispiel je eine Glasfaser oder je ein Bündel Glasfasern auf. Das über den Lichtleiter 18 zugeführte und durch die Blende 21 gestrahlte Licht kann über die beispielsweise mindestens eine Linse aufweisende Fokussiervorrichtung 22 zu einem auch im Gehäuse 3 angeordneten Dispersionselement 23 gelangen. Dieses ist beispielsweise durch ein lichtdurchlässiges Prisma gebildet, das zum Beispiel aus einem Saphir- oder Glasstück besteht.

Das vom Dispersionselement 23 beim Messen spektral zerlegte Licht kann zu einem auch im Gehäuse 3 angeordneten Lichtempfänger 25 gelangen, der zum Beispiel eine integrierte Schaltung besitzt und einen linienförmigen "Diodenarray" bildet. Jede von dessen Fotodioden dient als fotoelektrischer Wandler 25a zur Umwandlung von Licht in ein elektrisches Signal, das ein Mass für die Lichtintensität des zum betreffenden Wandler gelangenden Lichtes gibt. Während in der Fig. 1 nur einige wenige Wandler 25a gezeichnet sind, besitzt der Lichtempfänger 25 in Wirklichkeit mindestens 300 und vorzugsweise mindestens 500 Wandler 25a. Der Abstand von einander benachbarten Wandlern 25a beträgt zum Beispiel ungefähr 0,007 mm. Die Wandler können beim Betrieb als Signale elektrische Ladungen erzeugen. Die zum Lichtempfänger 25 gehörende, integrierte Schaltung kann dann ausgebildet sein, um die von den Wandlern erzeugten, elektrischen Ladungen durch einen Taktgeber zyklisch gesteuert abzutasten und durch eine sogenannte Ladungskopplung in eine Folge von elektrischen Spannungsimpulsen umzuwandeln.

Die Teile 5, 6, 7, 8, 21, 23, 25 können starr im Gehäuse 3 befestigt sein. Nötigenfalls können noch Stellmittel vorgesehen werden, mit denen der Hersteller der Einrichtung die Position des einen oder andern von diesen Teilen bei oder nach der Herstellung der Einrichtung justieren kann.

Die Einrichtung 1 weist noch eine Lichtumlenkvorrichtung 31 auf, die zum Beispiel derart am oder im Gehäuse 3 befestigt ist, dass der Sensor 14 bei der noch näher beschriebenen Referenzmessung an und/oder in ihr angeordnet werden kann. Die Lichtumlenkvorrichtung 31 besitzt zum Beispiel einen behälterförmigen Support 32, der auf einer Seite eine Öffnung und beim dieser gegenüberstehenden Grund einen metallischen Reflektor 33 hat. Der Support 32 ist derart ausgebildet, dass der Sensor 13 bei der Referenzmessung in die Öffnung gesteckt werden kann und dann auf einer Auflagefläche in Abstand vom Reflektor 33 aufliegt. Im übrigen kann der Sensor eventuell auch bei der Nichtbenutzung der Einrichtung vom Support 32 gehalten werden.

Die Teile 6, 7, 10, 13, 18, 21 bilden zusammen Lichtführungs- und Sensormittel 37. Wie noch näher erläutert wird, ermöglichen diese, von der Lichtquelle erzeugtes Licht wahlweise über das zu untersuchende Material oder über die Lichtumlenkvorrichtung 31 zum Dispersionselement 23 zu führen.

Die auch zur Einrichtung 1 gehörende Auswertungsvorrichtung 41 besitzt elektronische Schaltungsmittel 42, die beispielsweise einen Prozessrechner, Speichermittel und eine steuerbare Speisespannungsquelle für die Lichtquelle 5 aufweisen. Die Auswertungsvorrichtung 41 weist ferner Anzeige-und Registriermittel auf, die zum Beispiel durch eine Bildschirm- oder Flüssigkristall-Anzeigevorrichtung 43 und einen Drucker 44 gebildet sein können. Ferner sind noch manuell betätigbare Schalt- und/oder Stellorgane 45 vorhanden. Die Auswertungsvorrichtung 41 ist in der Fig. 1 schematisch durch einen ausserhalb des Gehäuses 3 gezeichneten Block dargestellt und kann in der Tat durch mindestens ein Gerät gebildet sein, das abgesehen von mindestens einem elektrischen Kabel mechanisch vom Gerät 2 und von dessen Gehäuse 3 getrennt ist. Es ist jedoch selbstverständlich auch möglich, die AuswertungsvorrIchtung 41 ganz oder teilweise im und/oder am Gehäuse anzuordnen.

Das Gehäuse 3 und die Auswertungsvorrichtung 41 können derart ausgebildet sein, dass sie problemlos von einer einzigen Person getragen werden können. Für die Durchführung einer Analyse kann die Einrichtung 1 dann in die Nähe des zu untersuchenden, sich beispielsweise in einem Bett befindenden Patienten gebracht werden.

In der Fig. 1 ist noch stark vereinfacht ein Ausschnitt aus der Haut des Körpers 51 des Patienten gezeichnet, dessen Blut untersucht werden soll. Im dargestellten Haut-Ausschnitt ist ein Blutgefäss 53 gezeichnet, das beispielsweise eine Arterie oder Arteriole bildet.

Nachdem das Gehäuse 3 und die Auswertungsvorrichtung 41 in der Nähe des Patienten aufgestellt sind, führt man für die Analyse eine Analysemessung, eine Referenzmessung und eventuell vor diesen Messungen noch Optimierungsmessungen durch. Für die Optimierungsmessungen und die Analysemessung wird der Sensor oder Messkopf an der Hautoberfläche des Körpers 51 angeordnet, wie es bei dem in der Fig. 1 mit unterbruchslosen Linien gezeichneten Sensor oder Messkopf 13 der Fall ist. Die Lichtstrahlungsmittel 15 und die Lichtaufnahmemittel 16 definieren auf der am Körper 51 anliegenden Seite des Sensors oder Messkopfs einen Lichtstrahlungsbereich bzw. einen Lichtaufnahmebereich. Die Lichtquelle 5 erzeugt dann ein Linienspektrum aufweisendes Licht. Dieses wird über die Fokussiervorrichtung 6, den Strahlenteiler 7 und den Lichtleiter 10 zu den Lichtstrahlungsmitteln 15 des Sensors oder Messkopfs 13 geführt und bei dessen Lichtstrahlungsbereich aus dem Sensor heraus und in den Körper 51 hineingestrahlt. Ein Teil dieses Lichtes wird dann durch Rückstreuung wieder aus dem Körper 51 heraus und beim Lichtaufnahmebereich in die Lichtaufnahmemittel 16 gestrahlt. Das aus dem bestrahlten Bereich des Körpers 51 zurückgestreute Licht wird in diesem unter anderem durch von Hämoglobinen verursachte Brechungs- und Absorptionsvorgänge beeinflusst. Das in die Lichtaufnahmemittel 16 gelangende Licht wird durch den Lichtleiter 18 in das Gerät 2 geleitet und über die Fokussiervorrichtung 21 in das Dispersionselement 23 gestrahlt. Dieses bewirkt eine spektrale Aufteilung des Lichtes und lenkt dieses in einer Ebene - nämlich in der Zeichenebene der Fig. 1 - abhängig von den Lichtwellenlängen in verschiedene Richtungen um. Das aufgeteilte Licht wird zum Lichtempfänger 25 und in dessen Wandler 25a gestrahlt. Da das von der Lichtquelle erzeugte Licht ein Linienspektrum besitzt, bildet das vom Dispersionselement 23 zum Lichtempfänger gestrahlte Licht nicht ein kontinuierliches Bündel, sondern separate, in verschiedene Richtungen gestrahlte Lichtstrahlenbündel oder - kurz gesagt - Lichtstrahlen, von denen jeder die Wellenlänge einer Linie des Linienspektrums hat. Jeder Strahl, der einer Linie des Linienspektrums oder des zur Benutzung vorgesehenen Teils des Linienspektrums entspricht, gelangt dann in mindestens einen der Wandler 25a, der eine elektrische Ladung erzeugt, deren Grösse ein Mass für die Lichtintensität des betreffenden Lichtstrahls gibt. Die den Lichtempfänger 25 bildende, integrierte Schaltung wandelt die durch Ladungen gebildeten, elektrischen Signale in eine Folge von elektrischen Spannungsimpulsen um und führt diese den elektronischen Schaltungsmitteln 42 der Auswertungsvorrichtung 41 zu. Die von den Wandlern 25a ursprünglich erzeugten Signale - d.h. elektrischen Ladungen - stellen die Lichtintensitätswerte in analoger Form dar. Diese werden jedoch schon vom Lichtempfänger 25 oder von den Schaltungsmitteln 42 digitalisiert.

Bei den nötigenfalls noch durchgeführten Optimierungsmessungen kann mittels der Stellvorrichtung 17 durch Verstellen der Lichtaufnahmemittel 16 der Abstand des Lichtaufnahmebereichs vom Lichtstrahlungsbereich stetig oder schrittweise verändert und gemäss einem vorgegebenen Abstandskriterium auf einen optimalen Wert eingestellt werden. Der Abstand kann zum Beispiel derart eingestellt werden, dass die Amplitude des im Takt der Herzfrequenz des Patienten pulsierenden Teils der bei einer vorgegebenen Spektrallinie gemessene Lichtintensität oder der Summe der bei mehreren vorgesehenen Spektrallinien gemessenen Lichtintensitäten den grösstmöglichen Wert hat.

Bei der nach allfälligen Optimierungsmessungen durchgeführten Analysemessung speichert der zu den Schaltungsmitteln 42 gehörende Prozessrechner dann für jeden Wandler 25a den von diesem bei der Analysemessung ermittelten Messwert in einen Speicher.

Für die Referenzmessung wird der Sensor oder Messkopf 13 von der Oberfläche des lebenden Körpers 51 entfernt und in die Öffnung des Supports 32 der Lichtumlenkvorrichtung 31 gesteckt. Der Sensor oder Messkopf 13 nimmt dann die strichpunktiert in der Fig. 1 gezeichnete Lage ein, in der seine zur Lichtabstrahlung und Lichtaufnahme dienende Seite dem Reflektor 33 zugewandt ist.

Die Intensität des bei den verschiedenen Messungen von der Lichtquelle erzeugten Lichtes kann zum Beispiel mit Hilfe des vom Strahlenteiler 7 zum Lichtsensor 8 abgezweigten Lichtes und den elektronischen Schaltungsmitteln 42 auf einen konstanten Wert geregelt werden.

Bei der Referenzmessung wird von der Lichtquelle 5 erzeugtes, das gleiche Linienspektrum wie bei der Analysemessung aufweisendes Licht zu den Lichtstrahlungsmitteln 15 geführt und von diesen zum Reflektor 33 gestrahlt. Mindestens ein Teil des von diesem reflektierenden Lichtes gelangt in die Lichtaufnahmemittel 16, wird dem Dispersionselement 23 zugeführt und von diesem gleich wie das dem Dispersionselement bei der Analysemessung zugeführte Licht spektral zerlegt sowie zum Lichtempfänger 25 gestrahlt. Die bei den verschiedenen Wandlern 25a bei der Referenzmessung gemessenen Messwerte der Lichtintensitäten werden analog wie bei der Analysemessung für jeden Wandler in einen Speicher der elektronischen Schaltungsmittel 42 gespeichert.

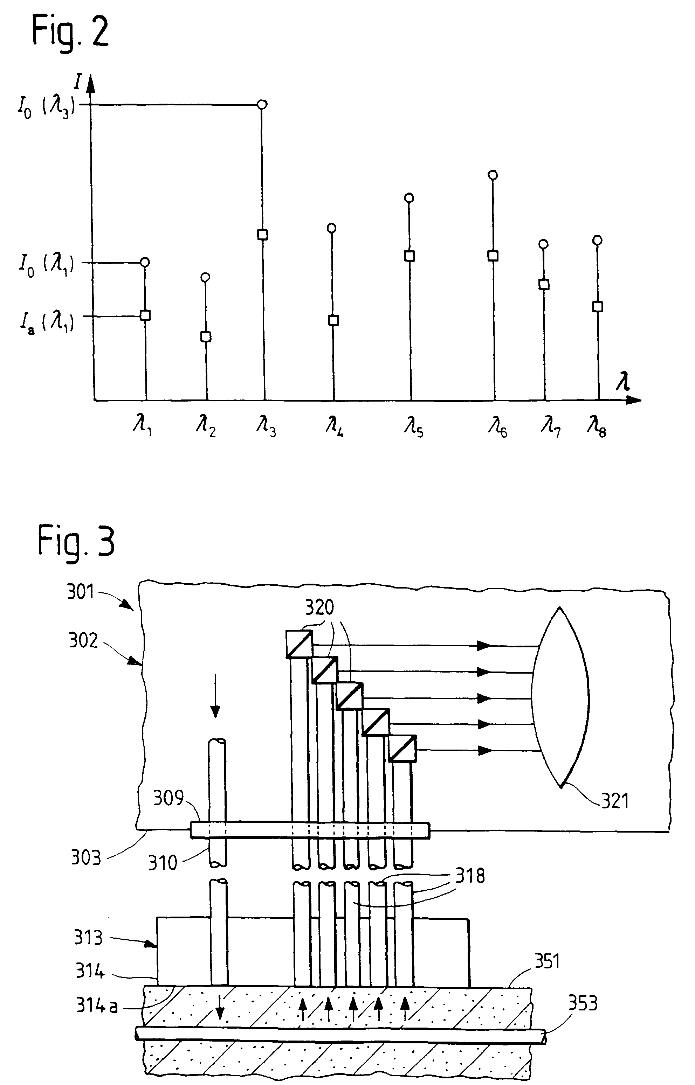

Die Auswertungsvorrichtung 41 kann in einem Auswertungsvorgang die bei der Analyse- und der Referenzmessung gemessenen Lichtintensitäten auswerten. Diese Auswertung wird anhand der Fig. 2 erläutert. In dieser ist auf der Abszisse die Lichtwellenlänge lambda und auf der Ordinate die Lichtintensität I aufgetragen. In der Fig. 2 ist schematisch ein Linienspektrum mit nur 8 Spektrallinien dargestellt, deren Wellenlängen die Werte lambda₁ bis Lambda₈ haben. In der Fig. 2 bezeichnen die kleinen Quadrate die bei der Analysemessung für die verschiedenen Wellenlängen gemessenen sowie gespeicherten Lichtintensitäts-Messwerte Iₐ und die kleinen Kreise die bei der Referenzmessung für die verschiedenen Wellenlängen gemessenen sowie gespeicherten Lichtintensitäts-Messwerte Iₒ. Zur Fig. 2 ist noch anzumerken, dass in dieser zur Verdeutlichung nicht ein reales, sondern ein fiktives Linienspektrum gezeichner wurde. Das tatsächlich für die Messungen benutzte Licht enthält normalerweise mehr als die in der Fig. 2 gezeichneten Spektrallinien. Im übrigen können in Wirklichkeit auch die Abstände der aufeinanderfolgenden Spektrallinien und Messwerte der Lichtintensitäten viel stärker voneinander verschieden sein, als es in der Fig. 2 gezeichnet ist.

Der zu den elektronischen Schaltungsmitteln 42 gehörende Prozessrechner ist ausgebildet und programmiert, um aufgrund von bekannten, vorgegebenen Wellenlängen der Spektrallinien des erzeugten Lichtes und aufgrund mindestens eines vorgegebenen Kriteriums bei der Referenzmessung, bei den allenfalls zum Einstellen des optimalen Abstandes der Lichtaufnahmemittel 16 von den Lichtstrahlungsmitteln 17 durchgeführten Messungen und bei der Analysemessung mindestens einigen der Wandler 25a gemäss einer festgelegten Vorschrift eine Wellenlänge zuzuordnen. Dabei werden mindestens denjenigen Wandlern Wellenlängen zugeordnet, welche das Licht der zur Verwertung vorgesehenen Spektrallinien empfangen. Dies ist auf verschiedene Arten möglich. Für jede nachfolgend beschriebene Methode für die Zuordnung von Wellenlängen zu Wandlern kann man bei oder nach der Herstellung der Einrichtung die Werte der Wellenlängen einiger oder aller von der Lichtquelle erzeugten und zur Verwendung bei der Auswertung der Messungen vorgesehenen Linien des Lichtspektrums in nicht löschbaren Speichern speichern.

Nun wird zuerst eine Methode zum Zuordnen von Wellenlängen zu Wandlern für den Fall erläutert, dass beim Messen mindestens einer der Endbereiche des von der Lichtquelle erzeugten Lichtspektrums zum Lichtempfänger gestrahlt wird. Die Einrichtung kann als Lichtquelle 5 zum Beispiel eine Niederdruck-Neonentladungslampe aufweisen und derart ausgebildet sein, dass der Lichtempfänger 25 mindestens Licht im Wellenlängenbereich von ungefähr 530 nm bis ungefähr 620 nm empfangen kann. Dann fällt beim Messen Licht des kurzwelligen, 12 Spektrallinien enthaltenden Endbereichs des Neonspektrums auf den Lichtempfänger. Dabei kann das Licht jeder Spektrallinie nur zu einem einzigen Wandler 25a oder zu einer Gruppe von zwei oder mehr einander benachbarter Wandler gelangen. Der Prozessrechner der zur Auswertungsvorrichtung 41 gehörenden Schaltungsmittel 42 kann nun zuerst das Licht der Spektrallinie mit der kürzesten Wellenlänge identifizieren und hierzu beim Messen denjenigen Licht empfangenden Wandler oder diejenige Gruppe von einander unmittelbar benachbarten sowie Licht empfangenden Wandlern ermitteln, der bzw. die sich am nächsten beim einen vorgegebenen Ende der Wandler-Reihe befindet. Die Auswertungsvorrichtung kann dann diesem Wandler bzw. dieser Wandler-Gruppe die kürzeste, 533,1 nm betragenden Wellenlänge des Neonlinienspektrums zuordnen. Danach kann die Auswertungsvorrichtung dem nächsten, Licht empfangenden Wandler oder der nächsten, Licht empfangenden Wandler-Gruppe ermitteln und diesen bzw. dieser die zweitkürzeste Wellenlänge des Neonlinienspektrum zuordnen. Die Auswertungsvorrichtung kann dann die restlichen, Licht empfangenden Wandler oder Wandler-Gruppen ermitteln und diesen entlang der Wandler-Reihe nacheinander die Wellenlängen der aufeinanderfolgenden Spektrallinien des Neonlinienspektrums zuordnen.

Die Auswertungsvorrichtung 41 kann zum Beispiel bei jeder Messung - also sowohl bei den allfälligen Optimierungsmessungen als auch bei jeder Analysemessung und jeder Referenzmessung - in der vorgängig beschriebenen Weise für jeden von einer der Spektrallinien des Neonspektrum zuordnen. Die Auswertungsvorrichtung kann jedoch auch ausgebildet sein, um nur bei einer der für eine Analyse durchgeführten Messungen - zum Beispiel nur bei der Referenzmessung - die zu bestimmten Wandlern gehörenden Wellenlängen in der beschriebenen Weise zu ermitteln. Die Auswertungsvorrichtung kann dann das Ergebnis dieser Ermittlung vorübergehend speichern und die für die Licht empfangenden Wandler oder Wandler-Gruppen ermittelten Wellenlängen den betreffenden Wandlern bzw. Wandler-Gruppen sowohl bei der Referenz- als auch bei der Analysemessung und eventuell auch bei allfälligen Optimierungsmessungen zuordnen. Des weiteren besteht die Möglichkeit, dass die Auswertungsvorrichtung nur einen Teil der Licht empfangenden Wandler oder Wandler-Gruppen direkt die Wellenlängen von Spektrallinien zuordnet und dann gemäss einer festgelegten Rechenvorschrift durch Interpolation und eventuell Extrapolation allen andern Wandlern eine Wellenlänge zuordnet.

Die vorgängig beschriebene Methode zum Zuordnen von Wellenlängen zu Wandlern kann in angepasster Weise auch in andern Fällen verwendet werden, in denen die Voraussetzung erfüllt ist, dass das von einem sich am nächsten beim einen Ende der Wandler-Reihe befindenden Wandler erfasste Licht eindeutig als Licht einer bestimmten Spektrallinie identifiziert werden kann.

Eine andere Möglichkeit zum Zuordnen von Wellenlängen zu Wandlern besteht darin, bei oder nach der Herstellung der Einrichtung für mindestens einen Teil der Spektrallinien die bei den Referenzmessungen zu erwartenden Lichtintensitäts-Sollwerte zu speichern. Dies kann zum Beispiel in der Form von in geeigneter Weise normierten, relativen Sollwerten geschehen, wobei etwa der grösste Sollwert auf den Wert 1 oder 100% normiert werden kann. Bei der Auswertung einer Referenzmessung kann der Prozessrechner dann den Wandlern Wellenlängen zuordnen, indem er die von den Wandlern gemessenen Messwerten mit den Sollwerten vergleicht oder die Messwertverhältnisse mit den Sollwert-Verhältnissen vergleicht.

Es sei ausdrücklich darauf hingewiesen, dass noch viele andere Methoden möglich sind, mit denen der Prozessrechner der Schaltungsmittel 42 mindestens denjenigen Wandlern 25a Wellenlängen zuordnen kann, die bei den Messungen Licht empfangenden, dessen Wellenlänge in dem zur Verwertung vorgesehenen Bereich liegt. Dabei hängt es auch vom Spektrum des benutzten Lichtes ab, wie die Zuordnung von Lichtwellenlängen zu Wandlern auf eine günstige Art durchgeführt wird.

Die Auswertungsvorrichtung 41 ist ferner ausgebildet, um aus den bei der Analysemessung und der dieser zugeordneten Referenzmessung ermittelten Messwerten für jede Spektrallinie eine Grösse auszurechnen und anzuzeigen und/oder zu registrieren, die ein Mass für die bei der Analysemessung erfolgte Lichtabsorption gibt. Der Prozessrechner kann hierzu zum Beispiel für jede Spektrallinie die Differenz der bei der Referenz- und der Analysemessung gemessenen Messwerte - also zum Beispiel die Differenz Iₒ (lambda₁) - Iₐ(lambda₁) ausrechnen. Der Prozessrechner kann diese Differenzen ferner noch normieren, indem er sie zum Beispiel durch den bei der betreffenden Spektrallinie gemessenen Messwert Iₒ dividiert und als Verhältniszahl oder Prozentwert darstellt. Diese Verhältniszahlen oder Prozentwerte können dann von der Flüssigkristall- oder Bildschirm-Anzeigevorrichtung 43 in Form eines Diagramms und/oder einer Tabelle angezeigt und vom Drucker 44 in mindestens einer der genannten Formen ausgedruckt werden. Es kann jedoch auch vorgesehen werden, dass der Prozessrechner für jede Spektrallinie den Absorptions - bzw. Extinktions-Koeffizienten ausrechnet und dass diese Koeffizienten in Form eines Diagramms und/oder in Form einer Tabelle angezeigt und/oder ausgedruckt werden.

Die zum Teil in der Fig. 3 gezeichnete Einrichtung 301 dient wie die Einrichtung 1 zur nicht-invasiven Analyse. Die Einrichtung 301 besitzt ein Gerät 302 mit einem Gehäuse 303 und einen Messkopf oder Sensor 313. Das Gerät 302 ist über an seinem Gehäuse angeordnete Durchführungs- und/oder Kupplungsmittel 309 und flexible Lichtleiter 310, 318 optisch mit dem Sensor oder Messkopf 313 verbunden. Der Lichtleiter 310 dient zum Zuleiten von Licht zum Sensor oder Messkopf 313, während die Lichtleiter 318 Licht von diesem zum Gerät 302 zurückleiten sollen. Der Sensor oder Messkopf besitzt einen Haltekörper 314 mit einer ebenen Grenzfläche 314a. Der Haltekörper 314 hält die einen Endabschnitte der Lichtleiter 310, 318 derart, dass deren Enden eine gerade Reihe bilden und die Endflächen der Lichtleiter 310, 318 oder an diesen angeordnete Endstücke und/oder Linsen vorzugsweise mindestens annähernd bündig mit der Grenzfläche 314a sind. Der Haltekörper 314 kann bei einer Analysemessung mit seiner Grenzfläche 314a an der Oberfläche eines lebenden Körpers 351 mit Blutgefässen anliegen, von denen eines gezeichnet und mit 353 bezeichnet ist. Das sich beim Haltekörper 314 befindende Ende des Lichtleiters 310 definiert und bildet einen Lichtstrahlungsbereich, bei dem bei einer Analysemessung Licht aus dem Lichtleiter 310 und dem Sensor heraus in den lebenden Körper 351 gestrahlt werden kann. Die sich im Haltekörper befindenden Enden des Lichtleiters 318 definieren und bilden sich in verschiedenen Abständen vom Lichtstrahlungsbereich befindenden Lichtaufnahmebereiche, bei denen im Körper 351 zurückgestreutes Licht in den Sensor und die Lichtleiter 318 gelangen kann.

Das Gerät 302 enthält eine nicht gezeichnete Lichtquelle, die beim Betrieb analog wie die Lichtquelle 5 des Gerätes 2 Licht erzeugen und über optische Komponenten in den Lichtleiter 310 strahlen kann. Das Gerät 302 enthält elektrisch steuerbare Lichtauswahlmittel 320. Diese besitzen zum Beispiel für jeden Lichtleiter ein elektrisch steuerbares, optisches Schaltorgan, von denen jedes einen optisch mit einem der Lichtleiter 318 verbundenen Lichteingang, einen Lichtausgang und mindestens einen elektrischen Steueranschluss besitzt. Die optischen Schaltorgane können durch Zuführen elektrischer Signale wahlweise in einen von zwei Schaltzuständen gebracht werden. In einem dieser Schaltzustände kann Licht vom Lichteingang zum Lichtausgang des betreffenden optischen Schaltorgans gelangen, während das letztere im andern Schaltzustand der Lichtdurchgang sperrt. Aus den Lichtausgängen der optischen Schaltorgane austretendes Licht kann über einen Lichtsammler 321 und durch eine nicht gezeichnete Blende zum Beispiel analog wie bei dem anhand der Fig. 1 beschriebenen Gerät 2 zu einem ebenfalls nicht gezeichneten Dispersionselement und von diesem zu einem Lichtauffänger gestrahlt werden.

Die elektrischen Steueranschlüsse der optischen Schaltorgane sind mit dem Prozessrechner einer der Auswertungsvorrichtung 41 entsprechenden Auswertungsvorrichtung verbunden. Bei einer Analysenmessung wählt der Prozessrechner der Auswertungsvorrichtung gemäss einem vorgegebenen Abstandskriterium mit Hilfe der Lichtauswahlmittel 320 das von einem Lichtleiter 318 oder eventuell von mehreren Lichtleitern 318 kommende Licht für die Verwertung bei der Analyse aus. Der Prozessrechner kann die Auswahl zum Beispiel gemäss einem ähnlichen Abstandskriterium treffen, wie es in bezug auf die in der Fig. 1 gezeichnete Einrichtung 1 für die Einstellung des Abstandes des Lichtaufnahmebereichs vom Lichtstrahlungsbereich des Sensors oder Messkopfs 13 mittels der Stellvorrichtung 17 beschrieben wurde.

Soweit vorgängig nicht anderes angegeben wurde, kann die Einrichtung 301 gleich oder ähnlich ausgebildet sein wie die Einrichtung 1 und auch ähnlich wie diese betrieben werden.

Die Einrichtung und die Verfahren zu deren Betrieb können noch in anderer Hinsicht modifiziert werden. Da der Lichtleiter 18 der Einrichtung 1 bereits den Querschnitt der heraus gestrahlten Lichtstrahlen begrenzt, kann man die Blende 21 der Einrichtung 1 eventuell weglassen. Ferner kann als Dispersionselement anstelle eines Prismas oder eines Beugungsgitters eine sogenannte Bragg-Zelle vorgesehen werden, die einen lichtdurchlässigen Körper besitzt, in welchem bei Analysen mit Ultraschall ein Beugungsgitter erzeugt wird.

## Patentansprüche

1. Einrichtung zur spektralfotometrischen Analyse eines Materials, mit einer Lichtquelle (5), welche als Spektrallichtquelle ausgebildet ist, so daß bei einer Analysemessung vom Material beeinflußtes, verschiedene Spektrallinien aufweisendes Licht zu verschiedenen fotoelektrischen Wandlern (25a) eines Lichtempfängers (25) gelangt, einer elektrisch mit dem Lichtempfänger (25) verbundenen Auswertungsvorrichtung (41), wobei der Lichtempfänger (25) zur Erzeugung von elektrischen Signalen ausgebildet ist, die ein Maß für die Lichtintensität von in seine Wandler (25a) gestrahltem Licht geben und einem Sensor (13, 313) zum Anliegen an der Oberfläche eines das zu analysierende Material enthaltenden Körpers (51, 351), mit Mitteln (10, 15, 16, 18, 310, 318), um von der Lichtquelle (5) erzeugtes Licht in den Körper (51, 351) zu strahlen und von diesem beeinflußtes sowie zurückgestreutes Licht aufzufangen, **dadurch gekennzeichnet**, daß ein Dispersionselement (23) zur spektralen Aufteilung des zurückgestreuten Lichtes vorgesehen ist, daß der Sensor (13, 313) ausgebildet ist, um bei einem Lichtstrahlungsbereich Licht aus dem Sensor (13, 313) heraus in den Körper (51, 351) zu strahlen und bei zumindest einem Lichtaufnahmebereich im Körper (51, 351) zurückgestreutes Licht aufzufangen, und daß der Abstand des Lichtaufnahmebereiches vom Lichtstrahlungsbereich verstellbar ist.

2. Einrichtung nach Anspruch 1, **dadurch gekennzeichnet**, daß der Sensor (313) ausgebildet ist, um bei mehreren, in verschiedenen Abständen vom Lichtstrahlungsbereich stehenden Lichtaufnahmebereichen im Körper zurückgestreutes Licht aufzufangen, und daß Lichtauswahlmittel (320) vorhanden sind, um mindestens einen der Lichtempfangsbereiche auszuwählen und das bei dem bzw. jedem gewählten Lichtempfangsbereich empfangene Licht dem Dispersionselement (23) zuzuführen.

3. Einrichtung nach Anspruch 1 oder 2, **dadurch gekennzeichnet**, daß die Lichtquelle (5) eine Niederdruck-Entladungslampe aufweist.

4. Einrichtung nach einem der Ansprüche 1 bis 3, **dadurch gekennzeichnet**, daß die Einrichtung ausgebildet ist, um bei einer der Analysemessung zugeordneten Referenzmessung mit der Lichtquelle (5) Licht mit dem gleichen Spektrum wie bei der Analysemessung zu erzeugen und ohne Beeinflussung durch ein zu analysierendes Material zum Dispersionselement (23) zu strahlen, und daß die Auswertungsvorrichtung (41) ausgebildet ist, um aufgrund von vorgegebenen Wellenlängen von Spektrallinien des Spektrums mindestens einem Teil der bei der Analysemessung und der Referenzmessung Licht empfangenden Wandler (25a) je eine Lichtwellenlänge zuzuordnen.

5. Einrichtung nach Anspruch 4, **dadurch gekennzeichnet**, daß die Auswertungsvorrichtung (41) ausgebildet ist, um mindestens für einige der Spektrallinien des von der Lichtquelle (5) erzeugten Lichtes sowohl bei der Analysemessung als auch bei der dieser zugeordneten Referenzmessung die Lichtintensität zu erfassen und für jede dieser Spektrallinien die Differenz zwischen der bei einer Analysemessung gemessenen Lichtintensität und der bei einer Referenzmessung gemessenen Lichtintensität zu ermitteln.

## Claims

1. Apparatus for spectrophotometric analysis of a material, with a light source 5 configured as spectral light source, such that light which is influenced by the material and has different spectral lines will arrive at different photoelectric transducers 25a of a light receiver 25 during an analysis measurement, and with an evaluation unit 41 that is electrically connected with said light receiver 25, the light receiver 25 being configured so as to generate electric signals providing a measure for the light intensity of light radiated into its transducers 25a, and a sensor 13, 313 to be applied to the surface of a body 51, 351 containing the material to be analyzed, with means 10, 15, 16, 18, 310, 318 to radiate light generated by the light source 5 into the body 51, 351 and to receive light influenced and scattered back by the latter, **characterized in that** a dispersion element 23 is provided for spectrally dispersing the backscattered light, and that the sensor 13, 313 is configured so as to radiate light from the sensor 13, 313 into the body 51, 351 at a light radiation region, and to pick up backscattered light from the body 51, 351 at at least one light receiving region, and further that the distance between light receiving region and light radiating region is adjustable.

2. Apparatus according to claim 1, **characterized in that** the sensor 313 is configured to pick up backscattered light from the body at several light receiving regions situated at different distances from the light radiation region, and that light selection means 320 are provided for the purpose of selecting at least one of the light receiving regions and transmitting the light received at the or each selected light receiving region to the dispersion element 23.

3. Apparatus according to claim 1 or 2, **characterized in that** the light source 5 is provided with a low pressure discharge lamp.

4. Apparatus according to any of claims 1 to 3, **characterized in that** the apparatus is configured such that the light which is generated by the light source 5 in the course of a reference measurement corresponding to the analysis measurement, has the same spectrum as that generated during the analysis measurement, and is radiated into the dispersion element 23 without being influenced by a material to be analyzed, and that the evaluation unit 41 is configured to assign one light wavelength each to at least some of the transducers 25a receiving light during the analysis and reference measurements, on the basis of defined wavelengths of spectral lines of the spectrum.

5. Apparatus according to claim 4, **characterized in that** the evaluation unit 41 is configured to detect the light intensity for at least some of the spectral lines of the light generated by the light source 5 during both the analysis measurement and the corresponding reference measurement, and to determine for each of said spectral lines the difference between the light intensity measured during an analysis measurement and the light intensity measured during a reference measurement.

## Revendications

1. Installation d'analyse photométrique spectrale d'une matière, comprenant une source lumineuse (5) en forme de source de lumière spectrale, de façon que pour une mesure d'analyse de matière, la lumière présentant différentes lignes spectrales influencées par la matière, arrive sur différents convertisseurs photoélectriques (25a) d'un récepteur photosensible (25), appartenant à un dispositif d'exploitation (41) relié électriquement au photorécepteur (25) générant des signaux électriques donnant une mesure de l'intensité lumineuse de la lumière émise par ces convertisseurs (25a) et un capteur (13, 313) pour appliquer à la surface d'un corps (51, 351) contenant la matière analysée, des moyens (10, 15, 16, 18, 310, 318) pour émettre la lumière générée par la source de lumière (5) dans le corps (51, 351) et recueillir la lumière réémise et influencée par ce corps,
caractérisée par
un élément de dispersion (23) pour la répartition spectrale de la lumière réémise, le capteur (13, 313) est réalisé pour que, dans une certaine plage d'émission de lumière, il (13, 313) émet de la lumière dans le corps (51, 351) et pour au moins une certaine plage de réception de lumière, reçoit la lumière réémise par ce corps (51, 351), la distance entre la plage de réception de lumière et la plage d'émission de lumière étant réglable.

2. Installation selon la revendication 1,
caractérisée en ce que
le capteur (313) est réalisé pour recevoir la lumière réémise dans le corps pour plusieurs plages de réception de lumière se trouvant à des distances différentes de la plage d'émission de lumière et des sélecteurs de lumière (320) sont prévus pour sélectionner au moins l'une des plages de réception de lumière et pour fournir à l'élément de dispersion (23) la lumière reçue dans la ou chaque plage choisie de réception de lumière.

3. Installation selon la revendication 1 ou 2,
caractérisée en ce que
la source lumineuse (5) est une lampe à décharge basse pression.

4. Installation selon l'une des revendications 1 à 3,
caractérisée en ce que
lors d'une mesure de référence associée à une mesure d'analyse, avec la source lumineuse (5), qui génère de la lumière avec le même spectre que pour la mesure d'analyse et la transmet à travers la matière analysée vers l'élément de dispersion (23) et à partir des longueurs d'ondes prédéterminées des lignes spectrales du spectre, le dispositif d'exploitation (41) attribue chaque fois une longueur d'onde de lumière à au moins une partie des convertisseurs (25a) recevant de la lumière pour la mesure de référence et la mesure d'analyse.

5. Installation selon la revendication 4,
caractérisée en ce que
au moins pour quelques lignes spectrales de la lumière générée par la source lumineuse (5), à la fois pour la mesure d'analyse et pour la mesure de référence qui lui est associée, le dispositif d'exploitation (41) détecte l'intensité lumineuse et, pour chacune des lignes spectrales, il détermine la différence entre l'intensité lumineuse mesurée lors de l'analyse de mesure et l'intensité lumineuse mesurée par une mesure de référence.
